(19) Europäisches Patentamt
European Patent Office
Office européen des brevets

(11) **EP 2 455 138 A1**

(12) **EUROPÄISCHE PATENTANMELDUNG**

(43) Veröffentlichungstag:
**23.05.2012 Patentblatt 2012/21**

(51) Int Cl.:
*A63B 24/00* *(2006.01)* *A61B 5/11* *(2006.01)*
*G06F 3/01* *(2006.01)* *G06K 9/00* *(2006.01)*
*A63B 71/06* *(2006.01)*

(21) Anmeldenummer: **11188476.3**

(22) Anmeldetag: **09.11.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **16.11.2010 DE 102010060592**

(71) Anmelder: **Fraunhofer-Gesellschaft zur Förderung der angewandten Forschung e.V.
80686 München (DE)**

(72) Erfinder:
• **Klose, Stefan
12529 Schönefeld (DE)**
• **John, Michael
10407 Berlin (DE)**

(74) Vertreter: **Gross, Felix
Patentanwälte Maikowski & Ninnemann
Postfach 15 09 20
10671 Berlin (DE)**

(54) **Trainingssystem, mobiles Endgerät und Trainingsverfahren für eine Person**

(57) Die Erfindung betrifft u.a. ein Trainingsystem für eine Person mit

a) einer mobilen Sensorvorrichtung (11, 12) zur Aufnahme von Bewegungsdaten und / oder Vitaldaten der Person,

b) einem Bildaufnahmemittel (3) für Bewegungen der Person, wobei

c) die Bewegungen der Person in Reaktion auf Bilder oder Signale erfolgen, die die Person auf mindestens einem Wiedergabemittel (4) sichtbar sind und

d) einer stationären Datenverarbeitungsvorrichtung (2), die das Wiedergabemittel (4) steuert, Bewegungsdaten und/oder Vitaldaten der Person empfängt, wobei

e) mit einem Analysemittel der stationären Datenverarbeitungsvorrichtung (2) ein Vergleich zwischen einem Ist-Zustand und einem Soll-Zustand der Bewegungen der Person durchführbar ist und

f) mit einem Feedbackmittel visuelle, haptische und/oder akustische Feedbacksignale automatisch in Abhängigkeit des Vergleichs zwischen Ist-Zustand und Soll-Zustand der Person zugänglich gemacht werden.

Die Erfindung betrifft auch ein Trainingssystem.

FIG 1

EP 2 455 138 A1

**Beschreibung**

Die vorliegende Erfindung betrifft ein Trainingssystem für eine Person nach Anspruch 1, ein mobiles Endgerät mit den Merkmalen des Anspruchs 9 und ein Trainingsverfahren für eine Person nach Anspruch 10.

Es ist seit langem bekannt, dass regelmäßiges Training des Körpers oder zumindest einzelner Körperpartien der Gesundheit förderlich ist. Dieses Training kann der Prävention, aber auch der Rehabilitation dienen.

Durch die alters- und gesundheitsbedingten Einschränkungen/ Funktionsstörungen des menschlichen Bewegungsapparates wie z. B. durch Abnutzungserscheinungen an Knochen oder Bändern, durch Übergewicht und Muskelabbau oder nach einem Unfall, einer Operation oder einer Erkrankung (z. B. Brüche, Bänderrisse, Schlaganfälle) können bestimmte Körperglieder bzw. -partien in ihrer Funktion und Beweglichkeit eingeschränkt sein.

Im Rahmen von Präventions- und Rehabilitationsmaßnahmen sollen diese betroffenen Körperregionen trainiert werden, um deren Funktion wiederherzustellen und/oder zu verbessern.

Dies geschieht bei schweren Fällen üblicherweise im Rahmen einer ca. dreiwöchigen stationären Rehabilitationsmaßnahme unter Führung von speziell ausgebildeten Therapeuten, begleitenden Nachsorgemaßnahmen wie IRENA oder für eine gesundheitliche Vorsorge im Rahmen der verfügbaren Präventionsprogramme.

Durch den immer höheren Altersdurchschnitt der Bevölkerung und die damit verbundenen Erkrankungen und Unfälle, steigt gleichzeitig die Zahl der orthopädischen Erkrankungen. Dies führt zu vermehrten stationären Aufenthalten und somit höheren Kosten bei den Krankenkassen.

Zurzeit erfolgt die Prävention oder Rehabilitation vor allem mit Übungen, die von einem Arzt oder Therapeuten ausgewählt, begutachtet, ausgewertet und korrigiert werden. Insbesondere bei der Auswertung und Korrektur können verschiedene Ärzte/Therapeuten zu unterschiedlichen Erkenntnissen kommen. Die Wirksamkeit einer Rehabilitationsmaßnahme ist somit abhängig von der begutachtenden Person.

Aus diesem Grund wurden in den letzten Jahren verschiedene rechner- und roboterunterstützte Verfahren entwickelt. In den meisten Fällen dient dabei die Hardware der Unterstützung bzw. Führung der geschädigten Körperregion. Die Hardware ist in der Regel auf eine Körperregion (untere Extremitäten, Hüfte, obere Extremitäten) spezialisiert.

Bekannte Verfahren für die oberen Extremitäten sind aus der DE202008011251U1, der EP000002156815A1, der WO002010108170A1, der WO002010092089A1 und der WO002010085476A1 bekannt.

Bekannte Verfahren für die Hüfte sind in der WO002010085111A2 beschrieben.

Bekannte Verfahren für die unteren Extremitäten sind aus der EP000002241302A1, der WO002010083542A1 und der WO002010054447A2 bekannt.

Die bekannten Lösungen setzen die Benutzung von teuren Robotern oder aufwändiger Hardware voraus, so dass diese Lösungen insbesondere nicht für die häusliche Rehabilitation geeignet sind. Auch werden die Personen gewissen Zwangsführungen unterworfen und die möglichen Freiheitsgrade werden eingeschränkt. Des Weiteren sind die Lösungen jeweils auf eine bestimmte Körperregion (z.B. Hand, obere Extremitäten, untere Extremitäten, Hüfte) spezialisiert, so dass eine ganzheitliche Betrachtung einer Person nicht möglich ist.

Durch den Einsatz von rechnerunterstützten Verfahren können diese Kosten gesenkt werden, da der Patient nach einer kürzeren stationären Behandlung in der Lage sein sollte, die Übungen selbstständig im heimischen Umfeld durchzuführen. Durch motivatorische Feedbackmechanismen werden Anreize zum eigenständigen Training gegeben, wodurch nachhaltige Erfolge erreicht werden.

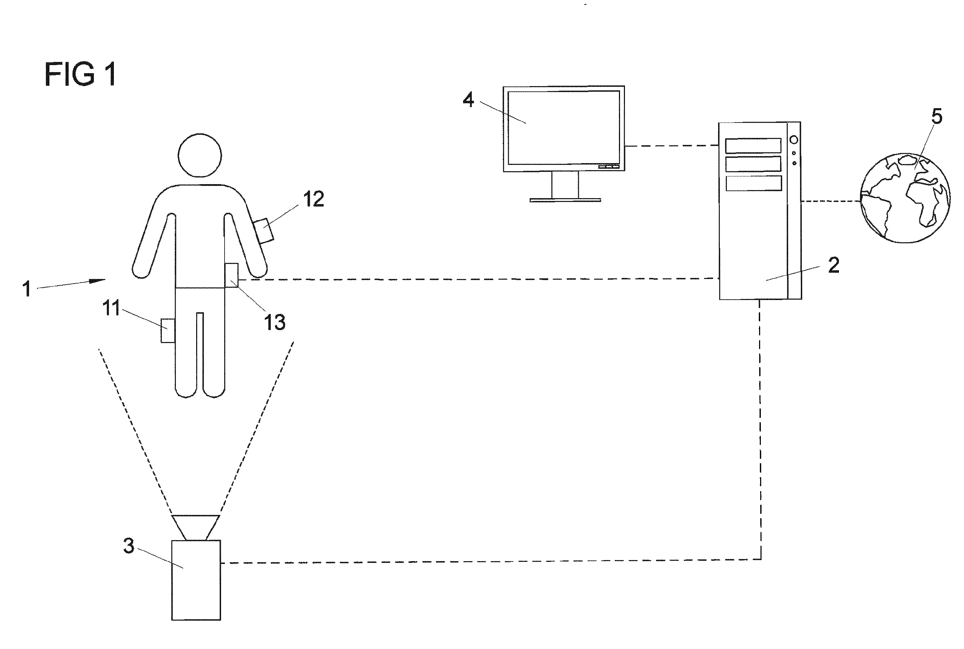

Bei der vorliegenden Erfindung handelt es sich um ein rechnergestütztes Trainingsverfahren und -system, das es ermöglicht, Vitaldaten- und Bewegungsdaten zusammenzuführen und zu analysieren. Das System weist Teilsysteme auf, die in Fig. 1 schematisch dargestellt sind.

Eine mobile Sensorvorrichtung 1 weist Sensoren 11, 12 auf, die in Sportgeräten (z.B. Nordic-Walking Stock) oder am Körper einer Person angeordnet sein können. Die z. B. Vital-, Druck und/oder Bewegungssensoren 11 sind mit einem mobilen Endgerät 13 gekoppelt, dass z. B. die Datenverbindung zu einer stationären Datenverarbeitungsvorrichtung herstellt. Die Person kann sich dabei im Außenraum oder in einem Innenraum aufhalten.

Das mobile Endgerät 13 erlaubt Feedbackmöglichkeiten, wenn die Person z.B. außer Haus ist. Eingeübte Trainingseinheiten können durch einen geeigneten Soll-Ist Vergleich vom mobilen Endgerät 13 bewertet werden, um der Person z.B. bei der Übung mit Nordic Walking Stöcken im Außenraum ein Feedback zu geben, ob die Übung effizient durchgeführt wird.

Die mobile Sensorvorrichtung 1 ist datentechnisch mit der stationären Datenverarbeitungsvorrichtung 2 verbindbar; in Fig. 1 durch eine gestrichelte Linie angedeutet. Die Datenverbindung kann dabei auch über das Internet erfolgen. Die stationäre Datenverarbeitungsvorrichtung 2 kann z. B. im häuslichen Umfeld oder in einer Therapieeinrichtung stehen. Wenn die Person z.B. nicht mit der stationären Datenverarbeitungsvorrichtung 2 verbunden ist, kann der Person Feedback-Informationen (was im Folgenden noch beschrieben wird) zur Verfügung stellen.

Ferner verfügt das Trainingssystem über mindestens ein Bildaufnahmemittel 3 (z.B. eine Webcam), das mit der stationären Datenverarbeitungsvorrichtung 2 gekoppelt ist.

Im Folgenden wird das Zusammenwirken der einzelnen Teilsysteme beschrieben.

Durch die Sensoren 11, 12 der Sensorvorrichtung 1 können an der Person z. B. folgende physikalischen Messwerte erfasst werden:

- Dreidimensionale Bewegung von Gliedmaßen im Raum
- Drücke (z. B. Anpressdruck beim Laufen) und Kräfte
- Dehnungen
- Vitaldaten (Körpertemperatur, Herzfrequenz, Puls, Sauerstoffsättigung, Feuchte der Haut)
- Ozonkonzentration, Luftfeuchtigkeit

Sowohl die mobile Sensorvorrichtung 1 wie auch die stationäre Datenverarbeitungsvorrichtung 2 können dazu folgende Komponenten aufweisen:

- Vitaldatenerfassung und -analyse
- Erfassung des Gemütszustands, z.B. über Bilderfassung des Gesichts
- Bewegungsdatenerfassung und -analyse
- Lokale Datenspeicherung
- Datenübertragung per Funk oder Kabel
- Mobile und/oder stationäre multimodale Feedbackvorrichtung 3 (haptisch, akustisch, visuell, z.B. auf einem Wiedergabemittel 4)
- Schnittstellen zu klinischen Dokumentationssystemen
- Integriertes Messagingsystem mit Videokonferenzlösung für Kontaktaufnahme von trainierender Person und Therapeut

Die einzelnen Sensoren 11, 12 können entweder einzeln oder im Verbund, d. h. in der Kommunikation mit anderen Sensoren 11, 12, sowohl mobil als auch stationär ausgewertet werden.

Sowohl die mobile Sensorvorrichtung 1, als auch die stationäre Datenverarbeitungsvorrichtung 2 zeichnen sich durch verschiedenen Formen der Feedbackgebung für die Person aus, die im Folgenden noch beschrieben werden.

Dazu werden in einer Phase des Trainings Bewegungsmuster der Person mit dem Bildaufnahmemittel 3 erfasst.

Eine Analysekomponente in der stationären Datenverarbeitungsvorrichtung 2 wertet dann die Daten über den Zustand von Bewegung und den Zustand von Vitaldaten (Ist-Zustand der Person) für die Person aus. Dabei werden die gemessenen Daten mit Solldaten verglichen und bestimmte Feedbacksignale automatisch bestimmt.

Hierfür werden verschiedene multimodale Feedbackgeber eingesetzt, wie z. B. ein Vibrator, ein akustischer Signalgeber und/oder eine grafische Anzeige von korrekten, idealen Sollbewegungsabläufen und Vitaldatenkurven bzw. den individuellen Bewegungsabläufen der Person. Die Sollbewegungsabläufe und Sollwerte der Vitaldatenkurven können sowohl auf der Basis generischer, medizinisch bewährter, d. h. anthropometrischer oder individueller Leistungsparameter der einzelnen Personen erhoben und analysiert werden.

Eine Ausführungsform des Trainingssystems besitzt eine Assistenzfunktion, mit dessen Hilfe es vorschlägt, wie die möglicherweise nicht idealen individuellen Bewegungsabläufe der Person einer therapeutisch idealen Bewegungsform angenähert werden können. Das Feedback kann dabei unmittelbar in Echtzeit oder zeitlich versetzt erfolgen.

Das System ist offen und kann durch hinzukommende Sensoren, z. B. Drucksensormatte, erweitert werden. Im Folgenden wird eine Ausführungsform des erfindungsgemäßen Verfahrens beispielhaft dargestellt.

In Fig. 1 ist schematisch angedeutet, dass die trainierende Person über die stationäre Datenverarbeitungsvorrichtung 2 Verbindung zu weiteren trainierenden Personen oder Therapeuten (symbolisiert als weltweite Community 5) herstellen kann. Diese Verbindungsmöglichkeit ist nicht nur für die trainierende Person interessant, sondern kann auch Forschungszwecken dienen.

Eine Ausführungsform des erfindungsgemäßen Trainingsverfahrens weist die folgenden Schritte auf: Initialisierung, Kalibrierung, Sensordatenakquisition, Sensordatenanalyse und Sensordatenfusion, Visualisierung, Live-Feedback, statistische Auswertung, Archivierung sowie Feedback.

Für die Durchführung der körperlichen Trainingsmaßnahmen, wie z. B. Rehabilitationsmaßnahmen wird ein Trainingssystem verwendet, wie es z. B. in Fig. 1 dargestellt ist.

Dabei wird mindestens ein Bildaufnahmemittel 3 zur Erfassung von Bewegungen der Person verwendet. Das Bildaufnahmemittel 3 kann dabei z. B. über eine oder mehrere Webcams verfügen. Auch ist es möglich, eine 3D-Kamera (z.B. Distanzerfassung zum Aufbau eines Tiefenbildes, Time-of-Flight) zu verwenden. Es können aber auch Infrarot-Kameras eingesetzt werden, um z. B. die Körpertemperatur der Person zu erfassen. Dies kann zusätzlich oder alternativ dazu verwendet werden, um festzustellen, wie sich die Temperatur der Person von der Temperatur der Umgebung unterscheidet. Die könnte eine Alternative zur Time-of-Flight Kamera sein.

Als Sensormittel 11, 12 werden z. B. körpernahe Sensoren wie z. B. Thermometer, Hautfeuchtesensoren, Pulsfühler, Blutdruckfühler, Dehnungssensoren, Beschleunigungsmesser oder Gyroskope verwendet. Damit können mechanische Belastungen und/oder Bewegungsmuster der Person aufgezeichnet werden. Die körpernahe Sensorik kann z. B. an oder in Kleidung angeordnet sein oder an Manschetten, Klettverbindungen, Klebeverbindung, Orthesen (d. h. einem medizinischen Hilfsmittel zur Unterstützung eingeschränkt funktionstüchtiger Körperteile) oder auf der Haut der Person. Grundsätzlich ist es auch möglich, die Sensormittel 11, 12 an oder in einer Endoprothese anzuordnen.

Ein Wiedergabegerät 4 (z. B. ein Bildschirm), das mit der stationären Datenverarbeitungsvorrichtung 2 gekoppelt ist, dient dazu, Trainingseinheiten und später dann das Feedback für die Person zu visualisieren und / oder für die Person mittels Audiodaten zur Gehör zu bringen. In der stationären Datenverarbeitungsvorrichtung 2 sind die Trainingseinheiten gespeichert und auch die vom Bildaufnahmemittel 3 aufgenommenen Daten werden darauf gespeichert. Die Trainingseinheiten können dabei von Ärzten, Therapeuten oder der Community (z.B. anderen Patienten) bereitgestellt und gespeichert werden.

Die stationäre Datenverarbeitungsvorrichtung 2 kann ein Computersystem sein, mit dem insbesondere die stationären Teilsysteme integriert sind. Es ist aber auch möglich, dass stationäre Teilsysteme in unterschiedlichen Komponenten angeordnet sind.

Vor der Durchführung des eigentlichen Trainingsverfahrens werden die Teilsysteme aufgebaut und in Betrieb genommen. Die Person stattet sich selbst oder wird durch einen Helfer mit der Sensorvorrichtung 1 ausgestattet.

Der erste Verfahrensschritt ist die Auswahl eines Soll-Modells für das durchzuführende Training für die Person. Das Soll-Modell repräsentiert den Soll-Zustand.

Die Auswahl kann durch die Person selbst oder einen Therapeuten vorgenommen werden. Das Soll-Modell für eine Trainingseinheit kann beispielsweise eine Videosequenz, eine 2D- oder 3D-Animation, ein 2D- oder 3D-Pfad und/oder eine andersartige Anordnung von virtuellen Interaktionsgegenständen aufweisen. Das Soll-Modell wird auf der Wiedergabevorrichtung 4 angezeigt. Während der Trainingseinheit hat die Person die Aufgabe, die Übung möglichst genau durchzuführen. Typischerweise weist die Trainingseinheit mehrere Wiederholungen auf.

In einem zweiten Verfahrensschritt werden die Sensoren 11, 12 kalibriert, die für die Erkennung der Bewegungen und Vitaldaten der Person während der Trainingseinheit eingesetzt werden, damit eine möglichst fehlerfreie Übungserkennung möglich ist.

Dies ist notwendig, da beispielsweise körpernahe Sensoren 11, 12 (z.B. Gyroscopes) relativ zur Umgebung (z.B. Erdschwerefeld, Erdmagnetfeld, lokales Magnetfeld) und nicht relativ zur Person angeordnet ist. Die Kalibrierung kann beispielsweise über die Einnahme einer definierten Pose Der Person - z. B. T-Pose - erfolgen).

Zur Kalibrierung von Gyroskopen kann die Person eine L-Pose mit dem linken bzw. rechten Arm einnehmen. Die Form der Pose erfolgt nach Abstimmung mit dem Therapeuten. Dabei ist die Körperfront der trainierenden Person parallel zum Bildaufnahmemittel 3 angeordnet.

Die spezifischen Kalibrierungsroutinen sind im stationären Datenverarbeitungsmittel 2 gespeichert. Dieses erkennt automatisch, welche Sensoren 11, 12 bei der Person installiert sind und bietet dazu auf dem Wiedergabemittel 4 die entsprechenden Kalibrierungsposen an, von denen zwei oben beispielhaft beschrieben wurden.

Die Ausrichtung der Person oder von Körperteilen der Person muss dabei nicht perfekt sein, d.h. Abweichungen von der Idealposition werden von der Bilderfassungssoftware in der stationären Datenverarbeitungsvorrichtung 2 toleriert.

Die korrekte Einnahme der Pose kann vom stationären Datenverarbeitungssystem 2 automatisch oder halbautomatisch erkannt werden oder manuell von einem Therapeuten oder von der Person ausgelöst werden. Im manuellen Fall z.B. per Sprachsteuerung.

Zum manuellen Auslösen muss die Person lediglich die Pose einnehmen und einen Kalibrierknopf drücken. Beim automatischen Auslösen wird zuerst das Gesicht der Person erkannt (per Bildaufnahmemittel, z. B. mittels Haar Cascade oder biometrischem Verfahren). Im erkannten Gesicht wird die Hautfarbe extrahiert. Anhand dieser Hautfarbe wird die Hand des zu therapierenden Arms gefunden. Somit ist ein Ankerpunkt für das weitere Erkennungsverfahren gefunden. Die Winkel von Unter- und Oberarm werden wie folgt bestimmt. Im ersten Schritt erfolgt eine Vordergrund/Hintergrund-Separierung. Dies kann beispielsweise über ein Gradientenverfahren oder die absolute Differenz von dynamischem Vordergrund und statischem Hintergrund erfolgen. Die Vordergrund/Hintergrund Separierung kann zusätzlich oder alternativ auch über die Z-Tiefe (d.h. der Abstand der übenden Person von einer 3D-Kamera erfolgen.

Das Ergebnis ist eine Vordergrundmaske. Mit Hilfe eines Skelettierungsverfahrens (z. B. Distance Transformation) wird die Anordnung der relevanten Körperglieder erkannt.

Somit kann automatisch erkannt werden, ob die Person die Kalibrierpose unter Berücksichtigung einer maximal erlaubten Abweichung eingenommen hat. Wenn das der Fall ist, ist die Kalibrierung in Bezug auf diesen Teil erfolgreich abgeschlossen.

Das Auslösen kann auch halbautomatisch über die extrahierte Hautfarbe erfolgen. Dabei nimmt die Person wiederum die Kalibrierpose ein und beobachtet die Aufnahme seines Oberkörpers auf dem Wiedergabemittel 4 (Spiegelanalogie). Sind die Person oder der Therapeut der Ansicht, dass die Pose korrekt ist, wird dies der stationären Datenverarbeitungsvorrichtung z. B. über eine Geste mitgeteilt. Eine einfache Möglichkeit ist das Schließen der Hand zur Faust. Im Fall z.B.: eines Schlaganfallpatienten kann dies z.B. auch das Öffnen einer Hand sein, da das Handschließen dieser Patientengruppe schwer fällt.

Die automatische, halbautomatische oder manuelle Auslösung kann in unterschiedlichen Phasen der Kalibrierung einzeln oder in Kombination angewendet werden.

Nach dem automatischen, halbautomatischen und/oder manuellen Auslösen der Kalibrierung werden die Gyroskope z. B. bezüglich der Ausrichtung von Ober- und Unterarm ausgerichtet (Alignment reset), d. h. bei der Verwendung der L-Pose ist die Orientierung des Unterarmgyroskops senkrecht, die des Oberarmgyroskops waagerecht. Bei anderen Posen erfolgt der Reset auf andere Art und Weise.

Für die Kalibrierung anderer Körperpartien, wie z. B. dem Oberkörper oder den Beinen, sind in analoger Weise, Kalibrierungsposen gespeichert, die bei Bedarf abgerufen werden können.

Für den Fall, dass eine oder mehrere Kameras als Bildaufnahmemittel 3 verwendet werden, ist es notwendig die radiale Bildverzerrung zu korrigieren. Dies geschieht bei Verwendung aktueller Hardware und Treiber üblicherweise schon treiberseitig.

Wird zur Übungserkennung beispielsweise ein Bildaufnahmemittel mit einem 3D-Stereo-Kamera-System verwendet, das zwei unabhängige Webcams aufweist, müssen diese auch zueinander kalibriert werden, so dass eine fehlerfreie 3D-Stereo-Rekonstruktion erfolgen kann. Dies kann beispielsweise über einen Kalibrierkörper oder einer Kalibriertafel erfolgen. Diese Kalibrierung kann einmal erfolgen und/oder vor jeder Durchführung einer Trainingseinheit. Die Kalibrierung kann auch unabhängig von den Trainingseinheiten durchgeführt werden. Es grundsätzlich auch möglich, ein kalibriertes System einzusetzen, bei dem die beiden Kameras in einem Gehäuse angeordnet sind.

In einem nächsten Verfahrensschritt erfolgt eine Sensordatenakquisition, -analyse und -fusion, was im Folgenden als Sensordatenverarbeitung bezeichnet wird

Die für die durchzuführende Trainingseinheit relevanten Sensoren 11, 12 sind eingeschaltet. Wie erwähnt, hat die Person die optionalen körpernahen Sensoren 11, 12 am Körper angebracht. Die Nutzung von unterstützenden Systemen (z. B. Orthese mit Servomotoren) ist abhängig vom Krankheitsbild der Person.

Die Art der Sensoren 11, 12 wird in Abhängigkeit von der durchzuführenden Trainingseinheit gewählt, d. h. für verschiedene Übungen werden unter Umständen unterschiedliche Sensoren 11, 12 benutzt.

Das Training wird gestartet, indem z. B. über ein Eingabemittel (beispielsweise Sprache, Gestik, Maus oder Tastatur) ein entsprechendes Signal an die stationäre Datenverarbeitungsvorrichtung 2 gegeben wird. Der Beginn der Trainingseinheit kann durch einen nach unten zählenden Countdown angekündigt werden oder sofort erfolgen. Sobald die Trainingseinheit gestartet wurde, werden die Daten sämtlicher Sensoren 11, 12 abgefragt und an die stationäre Datenverarbeitungsvorrichtung 2 weitergeleitet, wo sie ausgewertet werden. Die Weiterleitung der Daten kann sofort oder nach einer Zwischenspeicherung vorgenommen werden.

In Abhängigkeit der gewählten Trainingseinheit und der genutzten Sensoren 11, 12 wird aus den erfassten Daten ein partielles oder vollständiges Skelettmodell der Person generiert (Ist-Modell). Zur Generierung dieses Skelettmodells wird ein biomechanisches Modell genutzt, indem erfasste Sensordaten fusioniert werden. Dies geschieht aus Redundanzgründen, wenn bestimmte Sensoren 11, 12 gewisse Körperregionen nicht "sehen" können und zur Erhöhung der Genauigkeit. Die Art des generierten Skelettmodells. hängt dabei von der Art des Soll-Modells ab.

In einer vorteilhaften Ausführungsform können je nach Wunsch der Person während der durchgeführten Trainingseinheit die Daten der Soll-Übung, die Daten der Ist-Übung oder beide zusammen in geeigneter Weise visualisiert werden. Dabei kann es sich beispielsweise um Videos oder 3D-Animationen handeln. Für die anschließende statistische Auswertung wird der Übungsverlauf gespeichert.

Während der durchgeführten Trainingseinheit erfolgt durch ein Analysemittel (z. B. ein Computerprogramm auf der stationären Datenübertragungsvorrichtung 2) ein Vergleich zwischen Soll- und Ist-Zustand (d. h. zwischen Soll- und Ist-Modell) und die Generierung von Live-Feedbackdaten. Werden Abweichungen erkannt, wird die Person in geeigneter Weise darauf hingewiesen. Dies kann beispielsweise über Sprachausgabe oder die Einfärbung der betroffenen Körperregion auf dem Wiedergabemittel 4 erfolgen.

Nach Abschluss der Trainingseinheit erfolgt eine genauere Analyse des durchgeführten Trainings. Dabei wird beispielsweise der zeitliche und räumliche Verlauf von Soll- und Ist-Modell (d. h. zwischen Soll- und Ist-Zustand) analysiert. So kann detailliert auf Fehlstellungen oder zu schnell bzw. zu langsam durchgeführte Sequenzen hingewiesen werden.

Die durchgeführten Trainingseinheiten werden in einer Patientenakte gespeichert. Dabei kann es sich um eine lokale oder globale Datenbank handeln. Die gespeicherten Daten können für Vergleiche im Rahmen des Feedbacks genutzt werden.

Bei einigen, wenn nicht allen Arten der Datenspeicherung und / oder Übertragung werden vorteilhafterweise Verschlüsslungen verwendet, da Patientendaten grundsätzlich sensible Daten sind.

Im Anschluss an die Auswertung erfolgt das Feedback für die Person. Dies ist wichtig, um der Person Hinweise zur besseren Übungsdurchführung zu geben, aber auch um diesen - insbesondere beim heimischen Training - zu motivieren. Es gibt dabei zwei unterschiedliche Arten von Feedback: automatisch generiertes Feedback und Therapeuten-Feedback.

Das Therapeuten-Feedback kann per Videokonferenz (AV-Konferenz) erfolgen. Dabei wird die Patientenakte und die aktuell durchgeführte Übung (bzw. Übungen) mit einer automatisch generierten Analyse an einen Therapeuten weitergeleitet. Der Therapeut kann nun in einem persönlichen Gespräch mit der Person auf mögliche Fehlstellungen hinweisen und ihn ggf. motivieren. Vorteil dieser Methode ist, dass die Person menschliches Feedback erhält, ohne in die Klinik gehen zu müssen.

Bei der AV-Konferenz kann eine Aufnahme mit hoher Auflösung erfolgen (z.B. Full-HD, 1920x1080 Pixel). Die vorhandenen Bandbreiten für die Datenübertragung werden in der Regel aber vorteilhaft erscheinen lassen, dass nur eine verkleinerte Version der Bilder zum Therapeuten übertragen werden. Dabei kann der Therapeut einen besonders interessanten Bereich (Region of Interest ROI), wie z.B. einen geschädigten Arm auswählen. Diese ROI wird dann in hoher, insbesondere voller Auflösung übertragen. Damit können auch bei niedrigen Bandbreiten hochaufgelöste Aufnahmen genutzt werden.

Beim automatisch generierten Feedback wird eine Bewertung zwischen Soll- und Ist-Zustand (d.h. den Daten des Soll- und Ist-Modells) erzeugt. Dabei können beispielsweise die zeitlichen und räumlichen Abweichungen, aber auch Verbesserungen/Verschlechterungen gegenüber früher durchgeführten Übungen einfließen. Ein Vergleich kann beispielsweise auch mit anderen Personen (z. B. aus der eigenen Reha-Gruppe des stationären Aufenthalts) erfolgen, die dasselbe Krankheitsbild aufweisen. Dies ist in Fig. 1 durch die Community 5 dargestellt.

Dieser Ablauf soll im Folgenden anhand eines Ausführungsbeispiels für die Schlaganfallrehabilitation der oberen Extremitäten vertieft werden. Dabei wird angenommen, dass die Person durch den Schlaganfall beispielsweise Lähmungserscheinungen aufweist, die therapiert werden sollen. Durch das IT-unterstützte Training sollen Therapiefortschritte erzielt werden, aber auch Rückschritte und/oder der aktuelle Stand (im Vergleich zum Trainierenden oder anderen Vergleichspersonen) aufgezeigt werden.

Vor dem ersten Verfahrensschritt, wird als passendes Modul das Modul zur Therapie der oberen Extremitäten gewählt.

Im ersten Verfahrensschritt, der Kalibrierung, müssen die Gyroskope zur Bestimmung der räumlichen Lage und der Bewegung der Person eingestellt werden. Dazu muss die Person eine L-Pose mit dem linken oder rechten Arm einnehmen. Wie oben erwähnt, ist dies nur beispielhaft zu verstehen. Dabei ist die Körperfront der Person parallel zum Aufnahmemittel 3 angeordnet (Frontalaufnahme). Der zu therapierende Ober- und Unterarm befindet sich in der Körperfront-Ebene.

Der Oberarm ist in etwa parallel zum Boden und im rechten Winkel zum Oberkörper ausgerichtet. Der Unterarm ist in etwa senkrecht zum Boden und im rechten Winkel zum Oberarm ausgerichtet. Die Ausrichtung muss dabei nicht perfekt sein, d. h. Abweichungen von der Idealposition werden vom System toleriert. Die korrekte Einnahme der Pose kann vom System automatisch oder halbautomatisch erkannt werden oder manuell von einem Therapeuten oder von der Person ausgelöst werden, wie dies oben beschrieben wurde.

Im Folgenden wird die Anwendung der Kalibrierung auf die oberen Extremitäten beschrieben. Die Winkel von Unter- und Oberarm werden wie folgt bestimmt. Im ersten Schritt erfolgt eine Vordergrund/Hintergrund-Separierung. Dies kann beispielsweise über ein Gradientenverfahren oder die absolute Differenz von dynamischem Vordergrund und statischem Hintergrund erfolgen. Das Ergebnis ist eine Vordergrundmaske. Mit Hilfe eines Skelettierungsverfahrens (z. B. Distance Transformation) wird die Anordnung der relevanten Körperglieder erkannt. Somit kann automatisch erkannt werden, ob die Person die Kalibrierpose unter Berücksichtigung einer maximal erlaubten Abweichung eingenommen hat, wodurch die Kalibrierung ausgelöst wird.

Nach der Kalibrierung erfolgt die Sensordatenverarbeitung (Sensordatenakquisition, -analyse und -fusion). Da das Skelett der Person unter Umständen nicht immer erkannt werden kann (z. B. Verdeckung, identische Farbinformation), wird die relative Bewegung der kalibrierten Sensoren 11, 12 genutzt. Dazu wird ein biomechanisches Modell genutzt.

Das für diesen Fall bei der Kalibrierung verwendete biomechanische Modell basiert z. B. auf einfachen Annahmen über die Verhältnisse der einzelnen Körperglieder zueinander:

$$\text{Länge(Unterarm)} = 3 * \text{Breite(Faust)}$$

$$\text{Länge(Unterarm)} / 55 = \text{Länge(Oberarm)} / 45.$$

Diese generischen Verhältnisse können allerdings auch für jede Person separat exakt vermessen werden.

Damit werden bei der Kalibrierung der Sensorvorrichtung 1 automatisch Teile des Körpers erfasst und miteinander korreliert, insbesondere unter Ausnutzung relativer Bewegung von Sensoren 11, 12 und vorbestimmter geometrischer Proportionen des Körpers oder von Körperteilen.

Grundsätzlich können drei oder mehr unterschiedliche Sensoren 11, 12, die mit unterschiedlichen Verfahren arbeiten eingesetzt werden, um die gleiche Szene zu beobachten. Es kann mittels einer Mehrheitsentscheidung entschieden werden, welche Situation am wahrscheinlichsten beobachtet wurde.

Etwas weniger aufwändig ist es, wenn z.B. die Handposition über eine Webcam und Gyroscope erfasst wird. Wenn sich z.B. die Hand vor dem Gesicht befindet, ist sie in Echtzeit nur mit hohem Aufwand erkennbar. In einem solchen Fall kann dann eine Erkennung unter Verwendung der Gyroscope und des biomechanischen Models erfolgen. Befindet sich dann die Hand wieder außerhalb des Gesichtsbereiches, können wieder beide Sensorinformationen verwendet werden.

Das Training wird in diesem Ausführungsbeispiel mittels Faustgeste (oder alternativ mit dem Gegenteil davon, einer sich öffnenden Hand) gestartet. Die kalibrierten Sensoren 11, 12 und das daraus berechnete partielle Skelettmodell können visualisiert und aufgezeichnet werden. Bewegt die Person seinen Arm oder ihren Arm, verändert sich das erkannte Skelettmodell analog seinen Bewegungen. Dies kann u.U. für die Person angezeigt werden, in dem das Skelettmodell auf dem Wiedergabemittel 3 angezeigt wird. Dabei können z.B. bestimmte Körperpartien entsprechend dem Ergebnis der Analyse farbig eingefärbt sein.

Durch die bereits erwähnte Geste (alternativ oder zusätzlich durch Sprachsteuerung oder alternative Startknöpfe etc.) wird ein Pfad visualisiert, den die Person mit z.B. mit seiner Faust (oder der Hand oder einem anderen Körperteil) nachverfolgen soll. Dieser Pfad kann zufällig generiert sein oder vom Therapeuten (je nach Therapiefortschritt) gewählt werden. Dabei ist es beispielsweise wichtig, dass der Pfad über die Körpermitte der Person hinaus führt, da Schlaganfallpatienten mit einer Bewegung über die Körpermitte hinaus Probleme haben.

Wichtig kann dabei die Wiederholung sein. Ein Pfad soll zwar zufällig generiert werden, dann aber beispielsweise 100x verfolgt werden. Danach kann dann ein neuer Pfad generiert werden. Das bringt einen hohen Trainingseffekt.

Das Skelett der oberen Extremitäten und die Hand werden visualisiert. Dabei kann entweder nur das virtuelle Skelett visualisiert werden oder ein Overlay über die Kamera-Aufnahme der Person erfolgen.

Bewegt die Person den Arm, so wird die Position der einzelnen Armsegmente in Echtzeit aktualisiert. Die Person kann somit live mitverfolgen, wo sich sein Arm relativ zum nachzuverfolgenden Pfad befindet. Die aktuelle Handposition in Relation zum Pfad wird gespeichert.

Die Güte der Durchführung einer Übung wird in Abhängigkeit von der Abweichung vom Zentrum des Pfades bestimmt; es wird ein Live-Feedback gegeben. Der Pfad der Extremität kann in diesem Beispiel aus mehreren Regionen bestehen (gut, mittel, schlecht; sowie eine Orientierung: oberhalb, unterhalb, links oder rechts vom Pfad). In Abhängigkeit der Handposition kann die Güte beispielsweise über eine Ampel visualisiert werden (Grün = gut, gelb = mittel, rot = schlecht).

Nach Abschluss der Übung wird der verfolgte Pfad im Vergleich zum Soll-Pfad visualisiert. Des Weiteren erfolgt eine Analyse über die Gesamtgüte der durchgeführten Übung und eine entsprechende Gesamtbewertung. Darin fließen die Ausführungsdauer und die Güte der durchgeführten Übung ein. Kommt es in bestimmten Bereichen des Pfades zu starken Abweichungen, so wird die Person auf diese hingewiesen, so dass sie speziell solche Problemfälle trainieren kann.

Die verfolgten Pfade werden in einer Patientenakte gespeichert und können für Vergleiche im Rahmen des Feedbacks genutzt werden.

Anhand der statistischen Auswertung und der archivierten Daten erfolgt ein Feedback an die Person. Treten Verbesserungen (z. B. wochenweise) auf, so wird die Person gelobt. Es wird ihm vorgeschlagen den Schwierigkeitsgrad zu erhöhen. Treten keine Verbesserungen auf oder wird die

Übungsdurchführung schlechter, so wird der Person beispielsweise vorgeschlagen die Schwierigkeitsstufe zu verringern oder mit einem Therapeuten per AV-Konferenz in Kontakt zu treten.

Mit dem hier beschriebenen Trainingsverfahren und Trainingssystem ist es möglich, in einfacher Weise preiswerte Sensorik zum Einsatz zu bringen, wie z. B. Webcams, Gyroskope (Orientierungsmesser). Die Sensorik kann an verschiedenen Körperregionen angebracht werden oder diese "beobachten"; daher ist das Verfahren nicht auf eine Körperregion limitiert.

Das System kann sowohl in der stationären als auch häuslichen Rehabilitation eingesetzt werden.

Durch Sensordatenfusion optischer und körpernaher Sensorik kann die Genauigkeit erhöht und / oder eine verbesserte Redundanz erreicht werden.

Ferner ist es möglich, dass das Trainingsystem optional mit Zwangsführungs-/Unterstützungsgeräten (z. B. Orthesen) gekoppelt wird (z. B. initial mit Zwangsführung, bei Besserung des Zustandes der Person ohne diese).

Es ist möglich, unterschiedlich komplexe Trainingseinheiten für verschiedene Körperregionen in Abhängigkeit des Therapieziels und der gesundheitlichen Ausgangsposition der Person zu generieren.

Es ist möglich, eine Reihe von Feedbackmöglichkeiten in das System zu integrieren. Insbesondere ist ein "WhatYouSeeIsWhatYouGet-Feedback" (WYSIWYG) möglich, da eine Visualisierung der durchgeführten Trainingseinheit auf einem Wiedergabemittel 4 vorgesehen ist. Damit kann die Person einen selbstständigen Abgleich zwischen "Körpergefühl" und tatsächlich durchgeführter Übung durchführen (Analogie zu einem Spiegel).

Die Visualisierung der Trainingseinheit (Avatar oder Video) ist möglich, bevor sie durchgeführt wird, somit kann die Person die Trainingseinheit schon vor dem eigentlichen Training kennen lernen, insbesondere auch offline oder über das Internet.

Mit dem Soll-Ist-Vergleich zwischen durchgeführter Trainingseinheit und einer gespeicherten Trainingseinheit (z. B. Trainingsziel, Vergleichspersonen, eigener Stand vor einer Woche) kann eine differenzierte Bewertung erfolgen.

Mit der stationären Datenverarbeitungsvorrichtung sind ein automatisiertes Feedback und eine Bewertung über die Güte einer Trainingseinheit möglich, wobei diese anhand des Soll-Ist-Vergleichs generiert wird.

Es ist auch möglich, das Trainingssystem unter Einschaltung einer Videokonferenz zu verwenden. Die Person hat die Möglichkeit, sich mittels Videokonferenz mit einem Therapeuten oder befreundeten Rehabilitanden zu verbinden, dabei kann beispielsweise zusammen trainiert werden oder Tipps ausgetauscht werden. Es ist auch möglich, dass die stationäre Datenverarbeitungsvorrichtung 2 räumlich weit von den Personen getrennt ist. Die Personen führen die Trainingseinheiten außer Hause aus, wobei die Daten dann an die stationäre Datenverarbeitungsvorrichtung 2 übertragen werden.

Durch diese IT-unterstützte Rehabilitation sind Einschränkungen und Erfolge automatisierbar erkennbar und nachvollziehbar.

Das System kann Lebensbereich übergreifend eingesetzt werden für die gesundheitliche Prävention in der Freizeit (z. B. in der freien Natur oder in geschlossenen Räumen), im Sportverein, am Arbeitsplatz oder für die Rehabilitation in einer stationären Einrichtung wie z. B. einer Klinik oder Rehabilitationseinrichtung.

Insbesondere ist es mit dem Trainingsverfahren oder dem Trainingssystem gruppenartige Trainingsformen zu finden, auch wenn die einzelnen trainierenden Personen nicht alle in einem Raum anwesend sind. Die Personen sind z.B. in ihren Zimmern oder im Außenraum, können aber über die mobilen Endgeräte 13 gleichzeitig mit anderen trainierenden Person und / oder Therapeuten verbunden sein. So kann eine gegenseitige Kontrolle oder auch ein gegenseitiger Ansporn stattfinden.

Bezugszeichenliste

1    mobile Sensorvorrichtung

2    stationäre Datenverarbeitungsvorrichtung

3    Bildaufnahmemittel

4    Wiedergabemittel

5    Community

11    Sensor

12    Sensor

13    mobiles Endgerät

**Patentansprüche**

**1.** Trainingsystem für eine Personen mit

a) einer mobilen Sensorvorrichtung (11, 12) zur Aufnahme von Bewegungsdaten und / oder Vitaldaten der Person,
b) einem Bildaufnahmemittel (3) für Bewegungen der Person, wobei
c) die Bewegungen der Person in Reaktion auf Bilder oder Signale erfolgen, die die Person auf mindestens einem Wiedergabemittel (4) sichtbar sind und
d) einer stationären Datenverarbeitungsvorrichtung (2), die das Wiedergabemittel (4) steuert, Bewegungsdaten und/oder Vitaldaten der Person empfängt, wobei
e) mit einem Analysemittel der stationären Datenverarbeitungsvorrichtung (2) ein Vergleich zwischen einem Ist-Zustand und einem Soll-Zustand der Bewegungen der Person durchführbar ist und

f) mit einem Feedbackmittel visuelle, haptische und/oder akustische Feedbacksignale automatisch in Abhängigkeit des Vergleichs zwischen Ist-Zustand und Soll-Zustand der Person zugänglich gemacht werden.

2. Trainingsystem nach Anspruch 1, **dadurch gekennzeichnet, dass** der Soll-Zustand durch ein Soll-Modell einer Trainingseinheit für die Person definiert wird, wobei dies insbesondere durch eine gespeicherte Videosequenz, eine 2D- oder 3D-Animation, einen 2D- oder 3D-Pfad und/oder eine andersartige Anordnung von virtuellen Interaktionsgegenständen erfolgt.

3. Trainingsystem nach Anspruch 1 oder 2, **dadurch gekennzeichnet, dass** das mindestens eine Aufnahmemittel (3) eine Webcam, eine 3D-Kamera und/oder eine Infrarot-Kamera aufweist.

4. Trainingsystem nach mindestens einem der vorhergehen Ansprüche, **dadurch gekennzeichnet, dass** die Sensorvorrichtung mindestens einen Sensor (11, 12) zur Messung der Temperatur der Person, der Temperatur der Umgebung, von Dehnungen an Körperteilen der Person, von Beschleunigungen von Körperteilen der Person, der Hautfeuchte, der Feuchte der Umgebung, des Blutdrucks der Person, der Pulsrate der Person und/oder ein Gyroskop aufweist.

5. Trainingsystem nach mindestens einem der vorhergehenden Ansprüche, **dadurch gekennzeichnet, dass** das Wiedergabemittel (4) eine Trainingseinheit als Videosequenz und/oder als 2D oder 3D-Animation wiedergibt.

6. Trainingssystem nach mindestens einem der vorhergehenden Ansprüche, **dadurch gekennzeichnet , dass** die Datenübertragung zwischen einem mobilen Endgerät (13), der Person und der stationären Datenübertragungsvorrichtung über das Internet erfolgt.

7. Trainingssystem nach mindestens einem der vorhergehenden Ansprüche, **dadurch gekennzeichnet, dass** das mobile Endgerät (13) mittels eines Feedbackmittels Informationen für eine Person zur Verfügung stellt, auch wenn die mobile Sensorvorrichtung (11, 12) nicht mit der stationären Datenverarbeitungsvorrichtung (2)verbunden ist.

8. Trainingssystem nach mindestens einem der vorhergehenden Ansprüche, **dadurch gekennzeichnet, dass** zur Erkennung von Bewegungsdaten der Person und / oder der Kalibrierung die Informationen der Sensorvorrichtung (11, 12) und / oder ein biomechanisches Modell, insbesondere ein Skelettmodell verwendet wird.

9. Mobiles Endgerät besonders eingerichtet zur Verwendung in einem Trainingssystem gemäß den Ansprüchen 1 bis 8.

10. Trainingsverfahren für eine Person,
**dadurch gekennzeichnet, dass**

a) ein auf einer stationären Datenverarbeitungsvorrichtung gespeichertes Soll-Modell für eine Trainingseinheit einer Person ausgewählt wird,

b) vor der eigentlichen Aufnahme von Daten während der Trainingseinheit der Person eine Kalibrierung einer Sensorvorrichtung (11, 12) und/oder eines Aufnahmemittels (3) in Reaktion auf vorbestimmte Positionen und/ oder Bewegungen der Person erfolgt,

c) anschließend die Trainingseinheit der Person durchgeführt wird, wobei die stationäre Datenverarbeitungsvorrichtung (2) ein Wiedergabemittel (4) steuert, von dem die Person Informationen zur Trainingseinheit sieht, und

d) wobei die Sensorvorrichtung (11, 12) und/oder das Aufnahmemittel (3) die Bewegungen der Person, Umgebungsdaten und/oder Vitaldaten der Person erfassen und an die stationäre Datenverarbeitungsvorrichtung (2) übertragen, und

e) mit einem Analysemittel der stationären Datenverarbeitungsvorrichtung (2) ein Vergleich zwischen einem Ist-Zustand und einem Soll-Zustand der Bewegungen der Person erfolgt und

f) mit einem Feedbackmittel visuelle, haptische und/oder akustische Feedbacksignale automatisch in Abhängigkeit des Vergleichs zwischen Ist-Zustand und Soll-Zustand der Person zugänglich gemacht werden.

11. Trainingsverfahren nach Anspruch 10, **dadurch gekennzeichnet, dass** die Kalibrierung der Sensorvorrichtung (11, 12) automatisch Teile des Körpers erfasst und miteinander korreliert, insbesondere unter Ausnutzung relativer Bewegung von Sensoren (11, 12) und vorbestimmter geometrischer Proportionen des Körpers oder von Körperteilen.

**12.** Trainingsverfahren nach Anspruch 10 oder 11, **dadurch gekennzeichnet, dass** das Analysemittel beim Vergleich von Soll- und Ist-Zustand ein Modell des menschlichen Skeletts oder von Teilen eines Skeletts verwendet.

**13.** Trainingsverfahren nach mindestens einem der Ansprüche 10 bis 12, **dadurch gekennzeichnet, dass** die die Trainingseinheit zeitlich versetzt zur Auswertung der Sensordaten erfolgt.

# FIG 1

EP 2 455 138 A1

Europäisches
Patentamt

European
Patent Office

Office européen
des brevets

**EUROPÄISCHER RECHERCHENBERICHT**

Nummer der Anmeldung

EP 11 18 8476

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2010/084452 A1 (KONINKL PHILIPS ELECTRONICS NV [NL]; WANG JIM [CN]; LIU YUNQIANG [CN];) 29. Juli 2010 (2010-07-29) * Seite 3, Zeile 32 - Seite 8, Zeile 13; Abbildungen * ----- | 1-13 | INV. A63B24/00 A61B5/11 G06F3/01 G06K9/00 |
| X | WO 2008/055949 A1 (KHYMEIA S R L [IT]; FURLAN ROBERTO [IT]) 15. Mai 2008 (2008-05-15) * das ganze Dokument * ----- | 1-13 | ADD. A63B71/06 |
| X | WO 02/095714 A2 (LOESCHINGER JUERGEN [DE]) 28. November 2002 (2002-11-28) * Seite 3, Zeile 1 - Seite 6, Zeile 8; Abbildungen * * Seite 11, Zeile 25 - Seite 24, Zeile 32; Abbildung + * ----- | 1-13 | |
| X | WO 2010/085476 A1 (UNIV NORTHEASTERN [US]; SIVAK MARK [US]; HOLDEN MAUREEN K [US]; MAVROI) 29. Juli 2010 (2010-07-29) * Seite 12, Zeile 12 - Zeile 21 * * Seite 17, Zeile 22 - Seite 26, Zeile 5 * * Seite 43, Zeile 21 - Seite 45, Zeile 19; Abbildungen * ----- | 1-13 | RECHERCHIERTE SACHGEBIETE (IPC) A63B A61B G06F G06K |
| X | EP 1 123 686 A2 (WERK ROLAND DR MED DIPL BIOL [DE] WERK ROLAND DR MED DIPL-BIOL [DE]) 16. August 2001 (2001-08-16) * das ganze Dokument * ----- | 1-13 | |
| X,P | EP 2 383 021 A1 (TECHNOGYM SPA [IT]) 2. November 2011 (2011-11-02) * Absatz [0043] - Absatz [0112] * * Absatz [0234]; Abbildungen * ----- | 1-5,7-13 | |

Der vorliegende Recherchenbericht wurde für alle Patentansprüche erstellt

| Recherchenort | Abschlußdatum der Recherche | Prüfer |
|---|---|---|
| München | 17. April 2012 | Squeri, Michele |

**ANHANG ZUM EUROPÄISCHEN RECHERCHENBERICHT
ÜBER DIE EUROPÄISCHE PATENTANMELDUNG NR.**

EP 11 18 8476

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-04-2012

| Im Recherchenbericht angeführtes Patentdokument | Datum der Veröffentlichung | Mitglied(er) der Patentfamilie | | Datum der Veröffentlichung |
|---|---|---|---|---|
| WO 2010084452 A1 | 29-07-2010 | CN | 102292130 A | 21-12-2011 |
| | | EP | 2389760 A1 | 30-11-2011 |
| | | US | 2011273552 A1 | 10-11-2011 |
| | | WO | 2010084452 A1 | 29-07-2010 |
| WO 2008055949 A1 | 15-05-2008 | EP | 2089121 A1 | 19-08-2009 |
| | | US | 2009326341 A1 | 31-12-2009 |
| | | WO | 2008055949 A1 | 15-05-2008 |
| WO 02095714 A2 | 28-11-2002 | AU | 2002338767 A1 | 03-12-2002 |
| | | DE | 10124242 A1 | 28-11-2002 |
| | | EP | 1395968 A2 | 10-03-2004 |
| | | WO | 02095714 A2 | 28-11-2002 |
| WO 2010085476 A1 | 29-07-2010 | EP | 2389152 A1 | 30-11-2011 |
| | | WO | 2010085476 A1 | 29-07-2010 |
| EP 1123686 A2 | 16-08-2001 | DE | 10005955 A1 | 23-08-2001 |
| | | EP | 1123686 A2 | 16-08-2001 |
| EP 2383021 A1 | 02-11-2011 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202008011251 U1
- EP 000002156815 A1
- WO 002010108170 A1
- WO 002010092089 A1
- WO 002010085476 A1
- WO 002010085111 A2
- EP 000002241302 A1
- WO 002010083542 A1
- WO 002010054447 A2